Europäisches Patentamt

European Patent Office

Office européen des brevets

(11) Publication number: **0 129 938**
**B1**

⑫ . **EUROPEAN PATENT SPECIFICATION**

(45) Date of publication of patent specification: **19.10.88**

(21) Application number: **84200897.1**

(22) Date of filing: **21.06.84**

(51) Int. Cl.⁴: **G 01 N 23/04, H 05 G 1/64, A 61 B 6/14**

(54) **Dental X-ray examination apparatus.**

(30) Priority: **23.06.83 NL 8302233**

(43) Date of publication of application:
**02.01.85 Bulletin 85/01**

(45) Publication of the grant of the patent:
**19.10.88 Bulletin 88/42**

(84) Designated Contracting States:
**DE FR GB IT NL**

(56) References cited:
**EP-A-0 046 609**
**US-A-4 057 733**
**US-A-4 209 706**
**US-A-4 323 779**
**US-A-4 383 327**

**ORAL SURG., ORAL MED. & ORAL PATH., vol. 27, no. 4, April 1969, pages 475-491; J. VAN AKEN: "Optimum conditions for intraoral roentgenograms"**

(73) Proprietor: **N.V. Philips' Gloeilampenfabrieken**
**Groenewoudseweg 1**
**NL-5621 BA Eindhoven (NL)**

(72) Inventor: **Halm, Jan Hero**
**c/o INT. OCTROOIBUREAU B.V. Prof. Holstlaan 6**
**NL-5656 AA Eindhoven (NL)**
Inventor: **Post, Leonardus Henricus Johannes**
**c/o INT. OCTROOIBUREAU B.V. Prof. Holstlaan 6**
**NL-5656 AA Eindhoven (NL)**
Inventor: **van Otterdijk, Johannes Franciscus**
**c/o INT. OCTROOIBUREAU B.V. Prof. Holstlaan 6**
**NL-5656 AA Eindhoven (NL)**
Inventor: **van der Stelt, Paul Frans**
**c/o INT. OCTROOIBUREAU B.V. Prof. Holstlaan 6**
**NL-5656 AA Eindhoven (NL)**

(74) Representative: **Scheele, Edial François et al**
**INTERNATIONAAL OCTROOIBUREAU B.V. Prof. Holstlaan 6**
**NL-5656 AA Eindhoven (NL)**

## Description

The invention relates to an X-ray examination apparatus comprising an X-ray tube the anode of which is in the form of a tube extending from one end of said X-ray tube, a radiation window for said anode, an imaging X-ray detection means having an entrance screen, and means to displace said detection means with its screen with respect to said anode.

An X-ray examination apparatus of this kind is known from US—A—4,057,733. A dental apparatus described therein comprises an X-ray source with an anode pipe which can be inserted into the mouth of the patient for the formation of images, so that an intra-oral situation is provided for the radius focus and hence the source. For X-ray fluoreoscopy, the apparatus furthermore comprises an extra-oral radiation detection device which comprises an X-ray image intensifier tube. The X-ray image intensifier tube is arranged so as to be displaceable along the direction of a principal ray of the image forming X-ray beam and rotatable about the X-ray tube.

During the process of scanning a set of teeth by means of such an apparatus, freedom of movement is restricted. Moreover, because the radiation is incident at different angles for different members of a set of teeth, distortion occurs and a change-over of the scanning process from that for the upper set of teeth to that for the lower set of teeth and *vice versa* is comparatively difficult. Moreover, the introduction of such a range of movement also reduces the degree of reliability with respect to correct image formation.

It is an object of the invention to mitigate these drawbacks; to achieve this in accordance with the invention an X-ray examination apparatus of the kind set forth is characterized in that said displacement means is arranged to move said detection means such that the entrance screen moves in its own plane in two directions which are transverse to one another.

Because the radiation entrance screen in an X-ray examination apparatus in accordance with the invention is displaceable in a plane which is transverse with respect to the perpendicular to the entrance screen, by using, for example parallel techniques as described in Oral surg., Oral med. & Oral path., Vol. 27, No. 4, 1969, pp. 475—491, all the members of a set of teeth can be imaged without distortion while the position of the detector does not have an undesirable amount of freedom of movement.

In a preferred embodiment, the two lateral displacements are performed by means of, for example, screw-threaded spindle drives. If necessary, the drive may also include a level gear for accommodating an angle between a control spindle direction and the displacement direction. The control spindles are preferably provided with manually operable knobs. For a suitably reproducible setting, the apparatus may be provided with marks which are related to the displacements.

The radiation entrance screen in a preferred embodiment is formed by the entrance screen of, for example a 5 cm X-ray image intensifier tube, so that a comparatively high degree of brightness intensification can be achieved, thus allowing a comparatively low dose to be used for making radiographs as well as for fluoroscopy. The entrance screen preferably contains CsI as the entrance phosphor. The phosphor layer may be provided on the outer surface of an entrance window. The photocathode of the intensifier tube is then provided in a facing position on the inner surface of the window. Image scatter can be prevented by using a fiber optic plate for the manufacture of the entrance window. An exit window of the X-ray image intensifier tube can be read, for example by means of a television camera tube which is coupled thereto; however, a direct picture of the exit image can also be made, for example by means of an instant-print camera. On the other hand, a matrix of semiconductor radiation detectors may be associated with the exit screen, or the exit screen itself may comprise a matrix of electron-sensitive semiconductor detector elements. Alternatively, the X-ray image can itself be directly picked up by a television camera tube provided with an X-ray-sensitive entrance screen which then forms the entrance screen of the detection device and which contains, for example selenium as the radiation-sensitive material. For recording an image for analysis, a preferred embodiment includes a video memory and a television monitor; this embodiment may also include a V.H.U. (Video Hard-copy Unit) for making hard copies.

Because the entrance screen, and hence the imaging plane of the image, is always oriented parallel to the object plane in an apparatus in accordance with the invention, as has already been stated, no geometrical optical distortion occurs in the image. The main ray of the X-ray beam in principle extends transversely with respect to the tangent to the dental arch in the image. The half-shadow occurring in the image can be reduced by utilizing an X-ray tube in which a small focal spot is formed on the anode as the generating point for radiation. The cross-section of the X-ray beam can be adapted to the transverse dimension and the position of the entrance screen by means of a diaphragm.

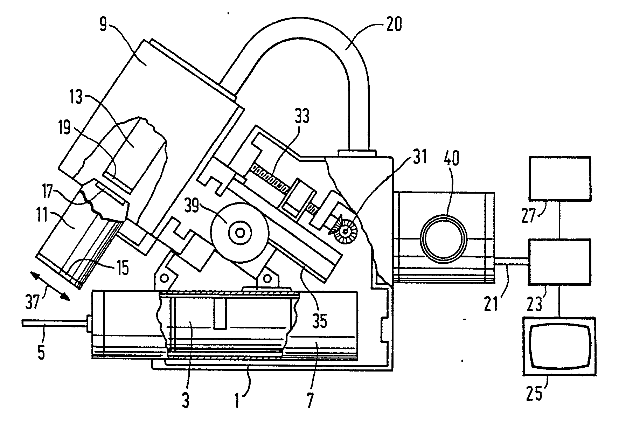

Some preferred embodiments in accordance with the invention will now be described by way of example, with reference to the drawing. The sole figure of the drawing shows an X-ray examination apparatus in accordance with the invention, comprising a first housing 1 which accommodates an X-ray tube 3 with a hollow anode pipe 5 and a high voltage generator 7, and a second housing 9 which in this case accommodates an X-ray image intensifier 11 and a television camera tube 13. The X-ray image intensifier tube comprises an entrance screen 15 with a phosphor screen and a photocathode, and an X-ray image exit screen 17 with, for example again a phosphor screen. *Via* an entrance window 19, the television camera tube is optically coupled to the exit window of the X-ray image intensifier tube, for example by means of a lens system or a fiber optical plate. The exit window may also consist of a fiber optical plate. *Via* a signal lead 21, video signals generated by the television camera tube are applied, *via* a video memory 23, to a television monitor 25 and, if desired, to a hard-copy unit 27.

Using a knob 31 and a spindle 33, the second housing can be displaced along a guide 35 with respect to the first housing in a direction in the plane of the drawing which is indicated by an arrow 37. *Via* a further spindle which is operated by means of an extenal knob 39, the second housing can be displaced in a direction which is transverse with respect to the plane of drawing, the entrance screen of the X-ray image intensifier tube then moving, as during the previous displacement, in a plane which is transverse with respect to a perpendicular to the entrance screen.

Instead of an X-ray image intensifier tube, use can be made of an X-ray sensitive screen from which an optical image, for example a photograph, can be recorded either directly or *via* an intermediate X-ray intensifier screen. In order to take optimum advanrage of the distortion-free imaging process, the entrance screen of the detection device is preferably flat. For example, for a 5 cm X-ray image intensifier tube it is quite satisfactory to use a flat entrance screen. If desired, the facility for image reduction provided by the X-ray image intensifier tube need not be employed enabling a 5 cm exit screen to be used. An adapted entrance screen of a camera tube may be coupled to such an exit screen or the exit screen can be projected in reduced form onto the entrance screen of the camera tube by optical means.

An advantageous embodiment utilizes a so-called SIT image intensifier tube as described in GB 1,538,561. Such an X-ray image intensifier tube includes an exit screen in which the electron image is directly converted, *via* a mosaic of semiconductor detection elements, into an image signal which can be read electronically.

For image storage for prolonged periods of time, a digital image memory may be connected to the apparatus, for example *via* an analog-to-digital converter. Also shown is a cable housing 38 to which a bush 40 si secured. By means of this bush, the apparatus can preferably be suspended so as to be translatable and rotatable from a ceiling support.

## Claims

1. An X-ray examination apparatus comprising an X-ray tube (3) the anode of which is in the form of a tube (5) extending from one end of said X-ray tube (3), a radiation window for said anode, an imaging X-ray detection means (11) having an entrance screen (15), and means (31, 39) to displace said detection means with its screen with respect to said anode, characterized in that said displacement means (31, 39) is arranged to move said detection means (11) such that the entrance screen (15) moves in its own plane in two directions which are transverse to one another.

2. An X-ray examination apparatus as claimed in Claim 1, characterized in that the detection means (11) comprises an X-ray image intensifier tube having a flat entrance screen (15).

3. An X-ray examination apparatus as claimed in Claim 1 or 2, characterized in that the X-ray detection means comprises an X-ray luminescent screen and a device for recording optical images generated in said screen.

4. An X-ray examination apparatus as claimed in Claim 1 or 2, characterized in that the X-ray detection means comprises an X-ray image intensifier tube with an exit screen in the form of a mosaic of electron-sensitive semiconductor elements.

5. An X-ray examination apparatus as claimed in any one of the preceding Claims, characterized in that the X-ray detection means comprises an instant camera (27) for forming directly usable hard-copy images of the optical images generated by the X-rays.

6. An X-ray examination apparatus as claimed in any one of the preceding Claims, characterized in that two controllable displacement mechanisms (31, 39) are provided for effecting the respective translatory displacement of the entrance screen (15) of the X-ray detection means (11) in two mutually perpendicular directions comprising two mutually perpendicular active movement mechanisms for the translatory movements of the detection entrance screen, each mechanism being provided with an externally operable control knob.

7. An X-ray examination apparatus as claimed in any one of the preceding Claims, characterized in that externally readable markings are provided which are respectively related to each of the displacements directions.

## Patentansprüche

1. Röntgenuntersuchungsgerät mit einer Röntgenröhre (3), deren Anode die Form eines Rohres (5) hat, das sich von einem Ende der Rötgenröhe (3) erstreckt, mit einem Strahlungsfenster für diese Anode, mit einem abildenden Röntgendetektionsmittel (11) mit einem Eintrittsschirm (15), und mit einem Mittel (31, 39) zum Verschieben des Detektionsmittels zusammen mit seinem Schirm in bezug auf die Anode, dadurch gekennzeichnet, daß das Verschiebungsmittel (31, 39) zum derartigen Verschieben des Dektionsmittels (11) angeordnet ist, daß der Eintrittsschirm (15) sich in der eigenen Ebene in zwei senkrecht zueinander verlaufenden Richtungen bewegt.

2. Röntgenuntersuchungsgerät nach Anspruch 1, dadurch gekennzeichnet, daß das Detektionsmittel (11) eine Röntgenbildverstärkerröhre mit einem flachen Eintrittsschirm (15) enthält.

3. Röntgenuntersuchungsgerät nach Anspruch 1 oder 2, dadurch gekennzeichnet, daß das

Röntgendetektionsmittel einen Röntgenleuchtschirm und eine Einrichtung zum Aufzeichen in diesem Schirm erzeugter optischer Bilder enthält.

4. Röntgenuntersuchungsgerät nach Anspruch 1 oder 2, dadurch gekennzeichnet, daß das Röntgendetektionsmittel eine Röntgenbildverstärkerröhre mit einem Austrittsschirm in Form eines Mosaiks aus elektronenempfindlichen Halbleiterelementen enthält.

5. Röntgenuntersuchungsgerät nach oder mehreren der vorangehenden Ansprüche, dadurch gekennzeichnet, daß das Röntgendetektionsmittel eine sofort ansprechbare Kamera (27) zur Bildung direkt verwendbarer Hardkopiebilder der mit Röntgenstrahlen erzeugten optischen Bilder enthält.

6. Röntgenuntersuchungsgerät nach einem oder mehreren der vorangehenden Ansprüche, dadurch gekennzeichnet, daß zwei steuerbare Verschiebungseinrichtungen (31, 39) zur Durch-führung der betreffenden translatorischen Ver-schiebung des Eintrittsschirms (15) des Röntgendetektionsmittels (11) in zwei senkrecht zueinander verlaufenden Richtungen vorgesehen sind und zwei senkrecht zueinander verlaufende aktive Bewegungseinrichtungen für die trans-latorischen Bewegungen des Detektionseintritts-schirms enthalten, wobei jede Einrichtung mit einer extern betätigbaren Steuertaste versehen ist.

7. Röntgenuntersuchungsgerät nach einem oder mehreren der vorangehenden Ansprüche, dadurch gekennzeichnet, daß extern lesbare Markierungen vorgesehen sind, die sich je auf jede der Verschiebungsrichtungen beziehen.

**Revendications**

1. Appareil d'examen à rayons X, comprenant un tube à rayons X (3) dont l'anode a la forme d'un tube (5) partant d'une extrémité du tube à rayons X (3), une fenêtre à rayonnement pour l'anode, un dispositif de détection à rayons X (11) pour la formation d'image comportant un écran d'entrée (15) et un dispositif (31, 39) pour déplacer le dispositif de détection avec son écran par rapport à l'anode, caractérisé en ce que le dispositif de déplacement (31, 39) déplace le dispositif de détection (11) de telle façon que l'écran d'entrée (15) se déplace dans son propre plan dans deux directions qui sont transversales l'une à l'autre.

2. Appareil d'examen à rayons X suivant la revendication 1, caractérisé en ce que le dispositif de détection (11) comprend un tube amplificateur d'image de rayons X comportant un écran d'entrée plat (15).

3. Appareil d'examen à rayons X suivant la revendication 1 ou 2, caractérisé en ce que le dispositif de détection de rayons X comprend un écran luminescent à l'égard des rayons X et un dispositif pour unregistrer des images optiques produites dans l'écran.

4. Appareil d'examen à rayons X suivant la revendication 1 ou 2, caractérisé en ce que le dispositif de détection à rayons X comprend un tube amplificateur d'image de rayons X avec un écran de sortie ayant la forme d'une mosaïque d'éléments semiconducteurs sensibles aux électrons.

5. Appareil d'examen à rayons X suivant l'une quelconque des revendications précédentes, caractérisé en ce que le dispositif de détection à rayons X comprend un appareil photographique à développement instantané (27) pour former des images de recopie directement utilisables à partir des images optiques produites par les rayons X.

6. Appareil d'examen à rayons X suivant l'une quelconque des revendications précédentes, caractérisé en ce que deux mécanismes de déplacement réglables (31, 39) sont prévus pour effectuer les déplacements de translation respectifs de l'écran d'entrée (15) du dispositif de détection à rayons X (11) dans deux directions mutuellement perpendiculaires, comprenant deux mécanismes de déplacement actifs, mutuellement perpendiculaires, pour les mouvements de translation de l'écran d'entrée de détection, chaque mécanisme étant pourvu d'un volant de commande pouvant être manipulé de l'extérieur.

7. Appareil d'examen à rayons X suivant l'une quelconque des revendications précédentes, caractérisé en ce que des repères lisibles de l'extérieur sont prévus et sont respectivement en rapport avec chacune des directions de déplace-ment.